# EUROPEAN PATENT SPECIFICATION

(11) **EP 0 608 004 B1**
(45) Date of publication and mention of the grant of the patent: **02.01.2003**
(21) Application number: 94103625.3
(22) Date of filing: 11.08.1987
(51) Int. Cl.: C12Q 1/68, G01N 33/574, G01N 33/68, C07H 21/00

(54) **Human DNA in the diagnosis of retinoblastoma**
Menschliche DNS zum Nachweis von Retinoblastoma
ADN humaine pour le diagnostic du rétinoblastome

(30) Priority: 11.08.1986 US 895163
(43) Date of publication of application: 27.07.1994
(62) Divisional of application: 87307095.7
(73) Proprietor: MASSACHUSETTS EYE & EAR INFIRMARY, Boston, MA 02114 (US); WHITEHEAD INSTITUTE, Cambridge, MA 02130 (US)
(72) Inventor: Dryja, Thaddeus P., Milton, MA 02186 (US); Friend, Stephen, Somerville, MA 02143 (US)
(74) Representative: Schlich, George William

(56) References cited:
- WO-A-84/01389
- US-A- 4 599 305
- SCIENCE, vol.223, no.4640, 9 March 1984, LANCASTER, PA US pages 1028 - 1033 A.L. MURPHREE ET AL.
- PROCEEDINGS OF THE NATIONAL ACADEMY OF SCIENCES OF USA., vol.82, no.6, 1985, WASHINGTON US pages 1795 - 1799 G.E.GALLICK ET AL.
- PROCEEDINGS OF THE NATIONAL ACADEMY OF SCIENCES OF USA., vol.82, no.10, 1985, WASHINGTON US pages 3400 - 3404 T.TANAKA ET AL.
- NATURE., vol.329, 15 October 1987, LONDON GB pages 642 - 645 W-H.LEE ET AL.

## Description

This invention relates to retinoblastoma nucleic acids, cells transformed with such nucleic acids, retinoblastoma polypeptides and antibodies against them, immunodiagnostic methods, compositions for treating neoplasms in humans, and methods of detection and treatment of a defective human gene related to cancer, in particular, retinoblastoma and osteosarcoma.

In M. Lalande *et al*, Cancer Genet Cytogenet 23: 151-157 (1986) the cloning of different DNA segments in human chromosomes has lead to a set of probes that might be useful in the diagnosis of human retinoblastoma.

Retinoblastoma is a neoplastic condition of the retinal cells, observed almost exclusively in children between the ages of 0 and 4 years. If untreated, the malignant neoplastic retinal cells in the intraocular tumor travel to other parts of the body, forming foci of uncontrolled growth which are always fatal. The current treatment for a retinoblastoma is enucleation of the affected eye if the intraocular tumor is large; for small intraocular tumors, radiation therapy, laser therapy, or cryotherapy is preferred. There is no known successful treatment for metastatic retinoblastoma. Hence, early diagnosis of retinoblastoma to allow treatment before the tumor spreads outside the eye is crucial.

Murphree et al., *Science,* 223:1028-1033 (1984), reviews the genetic inheritance of retinoblastoma, and the chromosomal evidence that supports the role of a diploid pair of suppressor alleles in the development of this tumor.

There is evidence that retinoblastoma is caused by the functional loss of both homologous copies of the retinoblastoma (Rb) gene. Thus, individuals carrying one defective allele of the Rb gene are genetically predisposed to the disease. Children who have had one eye affected by retinoblastoma or who are related to someone with retinoblastoma may be genetically predisposed and therefore at risk of developing the disease. These individuals routinely are tested for retinoblastoma every 2-3 months by an ocular examination procedure which requires placing the child under general anesthesia.

In accordance with one aspect of the invention, we provide purified nucleic acid comprising a human retinoblastoma gene, or a unique subregion thereof.

In accordance with a second, and alternative aspect of the invention, we provide a cell transformed with a nucleic acid according to the first aspect of the invention.

We provide, according to a third and alternative aspect of this invention, a retinoblastoma polypeptide comprising an amino acid sequence coded for by a nucleic acid according to the first aspect of the invention.

According to a fourth and alternative aspect of the invention, we provide a composition suitable for treating a human patient having a neoplasm, the composition characterized in comprising functional retinoblastoma polypeptide and a pharmacologically acceptable carrier.

There is further provided, according to a fifth and alternative aspect of the invention, an immunization-produced antibody capable of binding specifically to a retinoblastoma polypeptide according to the third aspect of this invention.

We further provide, in accordance with a sixth and alternative aspect of the present invention, an immunodiagnostic method of determining whether a neoplasm in a patient is associated with a genetic abnormality in a protein, said protein being characterized in that absence of a functional form of said protein is known to allow neoplasms to develop, said method comprising the steps of contacting a biological sample isolated from said patient with an antibody that binds specifically to said protein, and determining whether an immune complex forms between said antibody and said protein as an indication of whether the patient's neoplasm results from a deficiency in said functional form of said protein.

We describe the use of genetic material corresponding to a normal human retinoblastoma gene or a unique subregion thereof in the preparation of material for use in a method of screening human patients comprising comparing the DNA of said patients with the said gene or subregion.

Also described are vectors comprising genetic material corresponding to a normal retinoblastoma gene, or a unique subregion thereof.

We also describe a method of screening human patients by comparing the DNA of these patients with the isolated normal human retinoblastoma (Rb) gene or a unique subregion thereof (the term "unique subregion" means a DNA sequence found in the Rb gene and not elsewhere in the human genome). This comparison allows detection of defective Rb alleles in the patients, to determine whether these patients need continual monitoring by the conventional examination procedure. More importantly, this comparison will identify those patients who do not have a defective Rb allele and thus are not at risk of developing retinoblastoma and do not have to be examined by the conventional procedure.

Preferably, the comparison between the patient's DNA and the normal Rb gene involves testing the patient's DNA with the isolated Rb gene to detect either large deletions or, alternatively, small deletions or point mutations in the Rb locus. To test for large deletions in a patient's Rb allele, the patient's DNA preferably is analyzed by DNA hybridization using probes made from the isolated normal Rb gene.

Small deletions or point mutations preferably are detected by either of two techniques. The nucleotide sequences of the patients' Rb alleles and the normal Rb gene can be determined and compared for differences. Alternatively, the patient's DNA is probed with the normal Rb gene and any mismatches in the resulting heteroduplexes are identified.

The isolated normal human retinoblastoma gene can be used to produce the normal Rb gene product for protein therapy of individuals determined to have a defective Rb allele.

We describe a method of detecting the presence, in a tumor sample, of a protein the absence of which is associated with a distinct set of neoplasms. The method comprises producing an antibody to the Rb protein, contacting the antibody with the tumor sample, and detecting immune complexes as an indication of the presence of the protein in the tumor sample. If a tumor lacks the Rb gene product, no immune complexes will be found, and one may conclude that the tumor was the result of mutant Rb alleles. This limits the pathologic diagnosis to those tumors known to be caused by mutant Rb alleles, such as retinoblastoma, osteosarcoma, and some undifferentiated tumors of unknown cellular origin. A more exact categorization of pathologic diagnosis of human tumors will result.

In the drawings,
Fig. 1 is a pictorial representation of the autoradiogram from a Northern blot probed with p7H30.7R;
.Fig. 2 is a diagrammatic representation of the restriction map of the insert in the clone p4.7R;
Fig 3 is a pictorial representation of the autoradiogram from a Northern blot probed with p4.7R;
Fig. 4 is a diagrammatic representation of the vectors p2AR3.8 and p2AR0.9;
Fig. 5 is a diagrammatic representation of the mismatch detection technique;
Fig. 6 is a diagrammatic representation of an example denaturing gel used in mismatch detection.
Fig. 7 is the sequence of the normal Rb gene, with flanking regions.

### Isolation of the Normal Rb Gene

The genetic locus involved in causing retinoblastoma has been assigned to the q14 band of human chromosome 13 (Sparkes et al., Science 208:1042 (1980). A cDNA clone, p4.7R, from this region of DNA has been shown to carry Rb gene sequences. This clone was obtained by the following general techniques.

### Isolation of cDNA Clone p4.7R

The human DNA probe pH3-8, isolated from a human chromosome 13 lambda phage library (Lalande et al., 1984, Cancer Genet. Cytogenet. 13:283), was used in a chromosome walking technique to isolate and map 30 kb of genomic DNA surrounding the H3-8 sequence. One fragment generated by this technique, named p7H30.7R, was found to recognize a DNA sequence in the mouse genome as well as within human chromosome 13 (Dryja et al., 1986, Proc. Nat. Acad. Sci. USA in press). The homology of p7H30.7R to both human and mouse DNA suggested that p7H30.7R contained coding sequences of a structural gene.

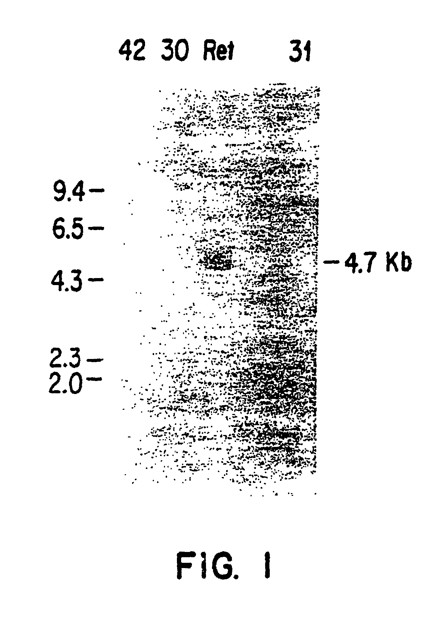

To test this possibility, p7H30.7R was radiolabeled and used to probe a Northern blot of RNA isolated from three retinoblastoma tumors (#42, #30, and #31) and an adenovirus 12-transformed human embryonic retinal cell line (Ret) (Vaessen et al., 1986, EMBO Journal 5:335). The p7H30.7R probe hybridized to an RNA transcript of approximately 4.7 kb from the retinal cell line, but did not hybridize to any RNA transcripts from the three tumor samples (Fig. 1).

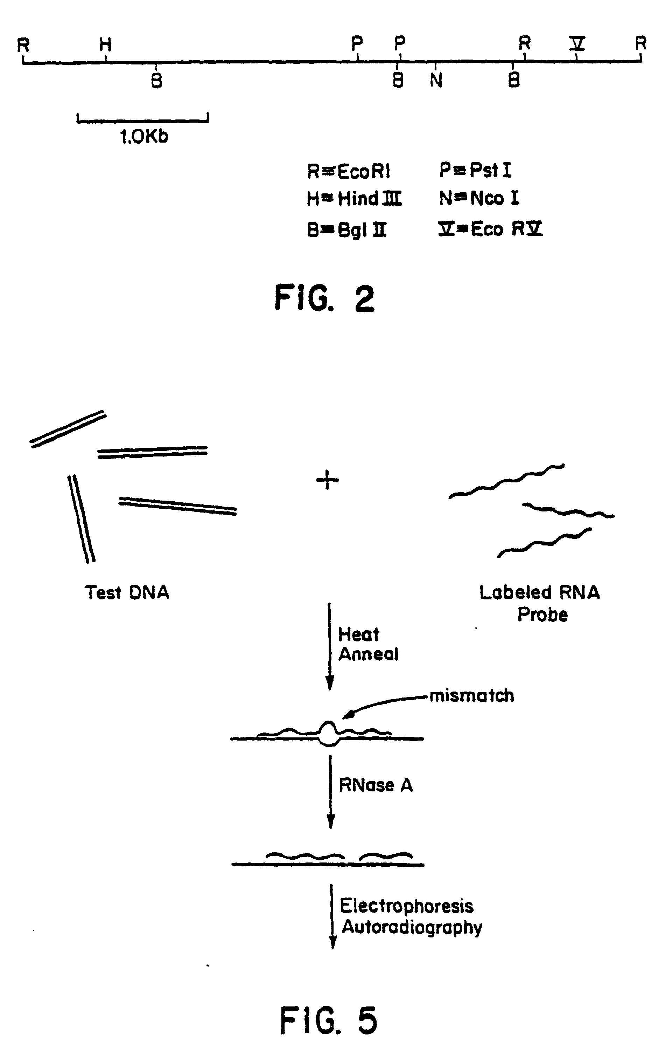

Subsequently, RNA isolated from the adenovirus-transformed retinal cell line was used to construct a cDNA library. This library was screened with the labeled p7H30.7R probe. Several cDNA clones were isolated which had similar restriction maps. The longest of these, p4.7R, contained 4.7 kb of genomic DNA. The physical map of p4.7R is shown in Fig. 2.

### Characterization of p4.7R

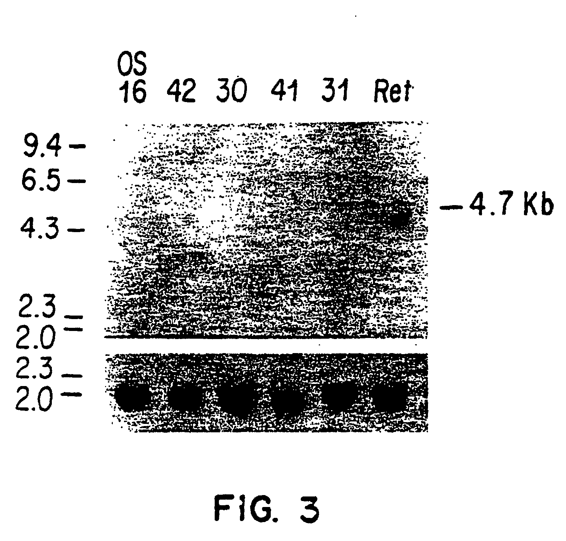

The p4.7R clone was used to screen RNA transcripts isolated from retinoblastomas (#42, #30, #41, #31), an osteosarcoma (#16), and the adenovirus-transformed retinal cells (Ret). As shown in Fig. 3, the p4.7R probe detected, in a Northern blot analysis of isolated RNA's, a transcript in the transformed retinal cells which is not present in the four retinoblastoma and one osteosarcoma cell samples. The bands at ∼ 2.0 kb were detected by reprobing the Northern blot, after washing, with a probe that detects rat tubulin (to demonstrate the presence of RNA in the blot).

The p4.7R clone also was used to screen genomic DNA. DNA was isolated from a set of tumors from 50 unrelated individuals, consisting of 40 retinoblastomas, 8 osteosarcomas, and 2 undifferentiated tumors of unknown cellular origin arising in patients with hereditary retinoblastoma. The isolated samples of DNA were digested with HindIII and analyzed by Southern blot hybridization using radiolabeled p4.7R as the probe. This analysis revealed three types of deviant patterns of the genomic DNA restriction fragments: totally absent fragments, representing apparent homozygous deletions; under-represented fragments, representing apparent heterozygous deletions; and fragments of altered size, reflecting either partial deletion or an alteration of a restriction site. At least 30% of the tumor DNA's exhibited one of these abnormalities. In comparison, Southern blot analysis of leukocyte DNA from 18 normal individuals showed a uniform pattern of restriction fragments.

The above results indicate that p4.7R detects the Rb gene. The deletion pattern in one osteosarcoma DNA sample provided particularly good evidence that p4.7R detects the Rb gene. This DNA sample is homozygous for a deletion that maps entirely within the p4.7R region. It is highly unlikely that the osteosarcoma phenotype arose due to a mutation independent of this deletion. Since the deletion is limited to the p4.7R region, this region must contain the Rb gene which, when mutated, produces non-functional Rb-encoded protein. The absence of functional Rb protein allows the neoplastic phenotype to develop.

### Use

The p4.7R sequences can be used to screen individuals for the presence of a mutated allele of the Rb gene. This screening procedure will allow individuals having a risk of developing retinoblastoma--because of family history or a previous incidence of retinoblastoma in one eye--to determine the need for routine testing by the current ocular examination procedure. Only if the screening procedure determines that the individual possesses a mutant Rb allele will the examination procedure need to be conducted on a regular basis. Those with two normal Rb alleles can discontinue examination, as the risk of developing retinoblastoma in an individual with two normal copies of the Rb gene is approximately 1 in 20,000, or 0.005%, compared to a risk of 80%-90% if an individual has an Rb allele containing a mutation sufficient to inactivate the allele. Thus, a substantial percentage of individuals who are currently examined regularly are not actually at a greater risk than the general population: neither a family history of nor a previous incidence of retinoblastoma is conclusive evidence that an individual has the genetic predisposition to the disease. Therefore, such individuals, actually carrying two normal copies of the Rb gene, have been repeatedly undergoing the expensive and traumatic ocular examination procedure needlessly.

The screening procedure can be of two major types: (1) testing an individual's DNA for deletions in the Rb locus large enough to interfere with hybridization to an Rb probe, and (2) testing an individual's DNA for small deletions or point mutations in the Rb locus.

In addition to screening, there is potential of providing protein therapy for those individuals determined to contain a mutant Rb allele and who therefore are at risk of developing retinoblastoma.

An additional use, as mentioned above, is in immunodiagnosis to determine, for example, whether a certain tumor is the result of an Rb gene abnormality. Since osteosarcomas and certain undifferentiated tumors can result from detectable lesions in the Rb gene, the immunodiagnosis can be used to aid in the diagnosis of such tumors.

Illustrative examples are given below.

### Example 1: Southern Blot Analysis

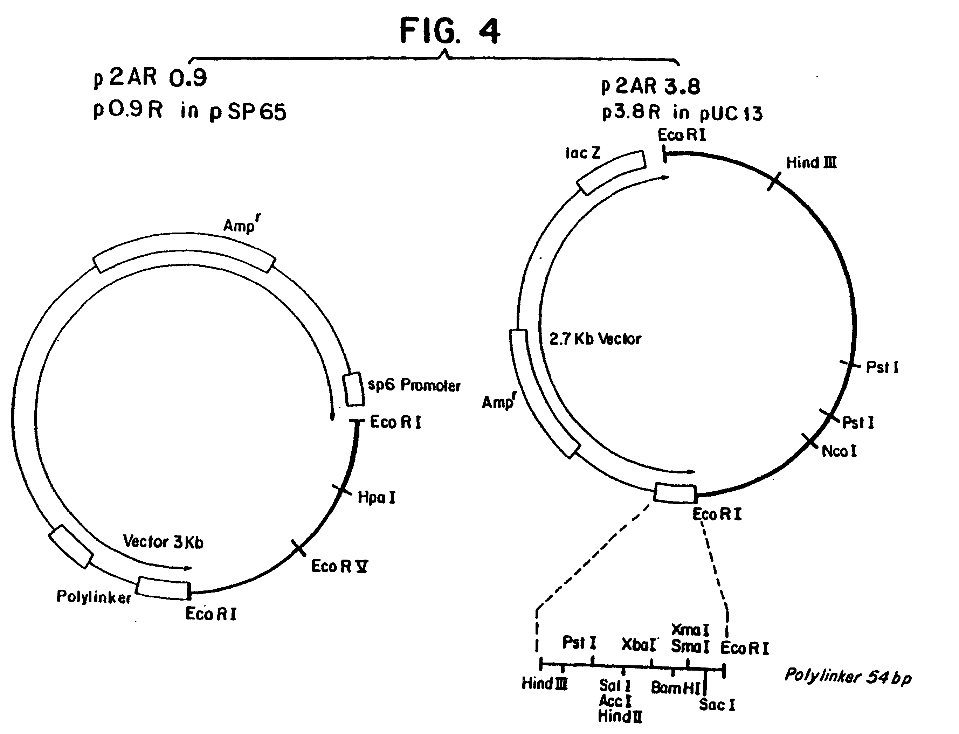

To detect large deletions in the Rb locus, a Southern Blot analysis is carried out on DNA obtained from an individual to be tested. The DNA is obtained from peripheral leucocytes or, if the patient has had a tumor in one eye, from the tumor. To examine leucocyte DNA, a 10 ml blood sample is obtained from the individual, and the genomic DNA is isolated from the leucocytes in the sample, according to standard techniques. This DNA is digested with a restriction endonuclease, e.g., HindIII, run on an agarose electrophoresis gel, and transferred to a nitrocellulose filter by blotting. The DNA on the filter is then probed with radiolabeled p2AR3.8 and, separately, p2AR0.9, containing subfragments from p4.7R obtained by EcoR1 digestion (Fig. 4); it is preferred to use two or more subfragments separately rather the entire p4.7R insert, in order to better define the location of any abnormalities detected. Autoradiograms of the probed filter give a restriction map of the Rb locus in the somatic or tumor DNA of the tested individual,

This restriction map then is compared with a control restriction map, determined by using the same restriction enzyme digestion and probe. A suitable control can be DNA obtained from the adenovirus-transformed retinal cell line or leucocyte DNA from a set of normal individuals. If the tested individual has an Rb allele containing a significantly large deletion, the above restriction map of his DNA, compared with the control, will contain an additional band or bands, and/or a band or bands that have lost 50% of their intensity, caused by a change in the size, or total elimination, of one or more restriction fragments by the deletion in one allele at the Rb locus.

Thus, this screening procedure by Southern analysis will detect the existence of non-functional Rb alleles which have large deletions. If this analysis indicates that the tested DNA from an individual has a restriction map different from the control map, there is a great probability that the individual contains a non-functional, mutant Rb allele. The individual must be monitored closely thereafter for the development of retinoblastoma.

If the test restriction map appears identical to the control, a different screening procedure can be performed on the individual's DNA to determine if the individual contains an Rb allele having a small deletion or point mutation, which is sufficient to inactivate the allele but not to prevent hybridization with a probe. This screening procedure is described in the following example.

### Example 2: Rb Locus Fine Structural Analysis

To examine an individual's DNA for small deletions or point mutations in the Rb locus, both homologs of the Rb gene from the individual preferably are cloned. The cloned alleles then can be tested for the presence of sequence differences from the normal allele, represented by p4.7R, by one of the following two methods: (1) the nucleotide sequence of both the cloned alleles and p4.7R are determined and then compared, or (2) RNA transcripts from p4.7R are hybridized to single stranded whole genomic DNA from an individual to be tested, and the resulting heteroduplex is treated with RNase A and run on a denaturing gel to detect the location of any mismatches. In more detail, these methods are carried out as follows:

### (1) Cloning Rb alleles

The alleles of the Rb gene in an individual to be tested are cloned using conventional techniques. A common method, for example, employs the bacteriophage vector EMBL3 (Frischauf et al., 1983, J. Mol. Biol. 170:827). A 10 ml blood sample is obtained from the individual, and the genomic DNA is isolated from the cells in this sample. This DNA is partially digested with MboI to an average fragment size of approximately 20 kb. Fragments in the range from 18-21 kb are isolated. The resulting MboI-ended fragments are ligated into the EMBL3 vector DNA which has been completely digested with BamHI, treated with alkaline phosphatase, and heated to 68°C for 10 minutes to disrupt the cohesive ends. This ligation mix is used in an in vitro lambda packaging reaction, and the packaged phage are amplified by growing a plate stock. [This cloning technique is described generally in Maniatis et al., Molecular Cloning: A Laboratory Manual', Cold Spring Harbor Publications, pp 256-293 (1982).]

Approximately 5 x 10⁵ pfu from this plate stock are used to infect 3 ml of E. coli cells at ∼ 1.5 x 10⁹ cells/ml in 0.01M MgSO₄, and the infection mix is incubated at 37°C for 20 minutes. 65 ml melted top agar at 47°C is added, and the mixture is plated onto ten 150 mm plates containing freshly poured and dry bottom agar. The agar plates are incubated until the plaques reach a diamter of ∼ 1.5 mm and are just beginning to contact one another (approximately 10-12 hours).

Duplicate circular nitrocellulose filters (Millipore HAWP) are placed gently on the surface of each agar plate to bind the bacteriophage DNA. The filters are carefully removed after 1 minute, placed into a denuturing solution (1.5m NaCl, 0.5M NaOH) for 30 seconds, neutralized for 5 minutes (1.5M NaCl, 0.5M Tris-Cl pH 8.0), and dried under vacuum at 80°C for 2 hours.

These nitrocellulose filters then are probed with radiolabeled p4.7R by hybridization and autoradiography. Plaques which show hybridization to the p4.7R probe are plague-purified and rescreened according to the above procedure. Positive plaques from the rescreening are isolated and used to prepare DNA putatively containing Rb alleles from the individual.

The MboI genomic inserts in these isolated EMBL3 vector DNA samples are tested for the location of the sequences homologous to p4.7R by Southern analysis. DNA samples containing the entire Rb gene region are selected, and the appropriate restriction fragments containing the Rb gene from these samples are subcloned into a suitable vector, such as pUC9. These subclones thus contain copies of one or both Rb alleles from the DNA of the individual to be tested. To determine if both alleles are represented, the initial phage isolates are tested for the existence of restriction polymorphism. These subcloned alleles are then examined for differences from p4.7R by one of the following techniques.

### (2) Sequence Comparison

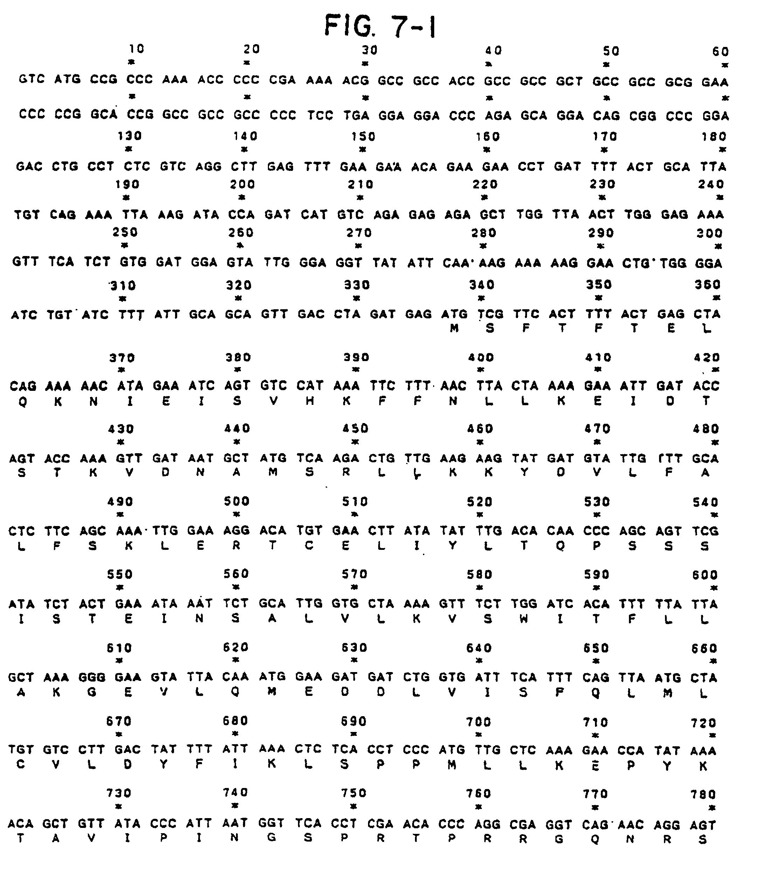

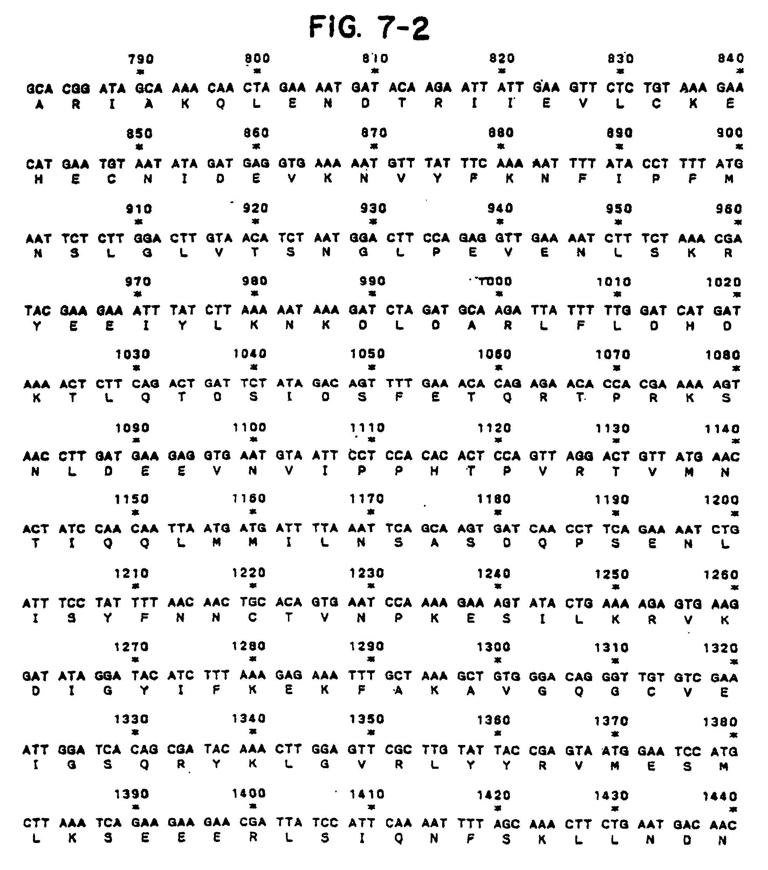

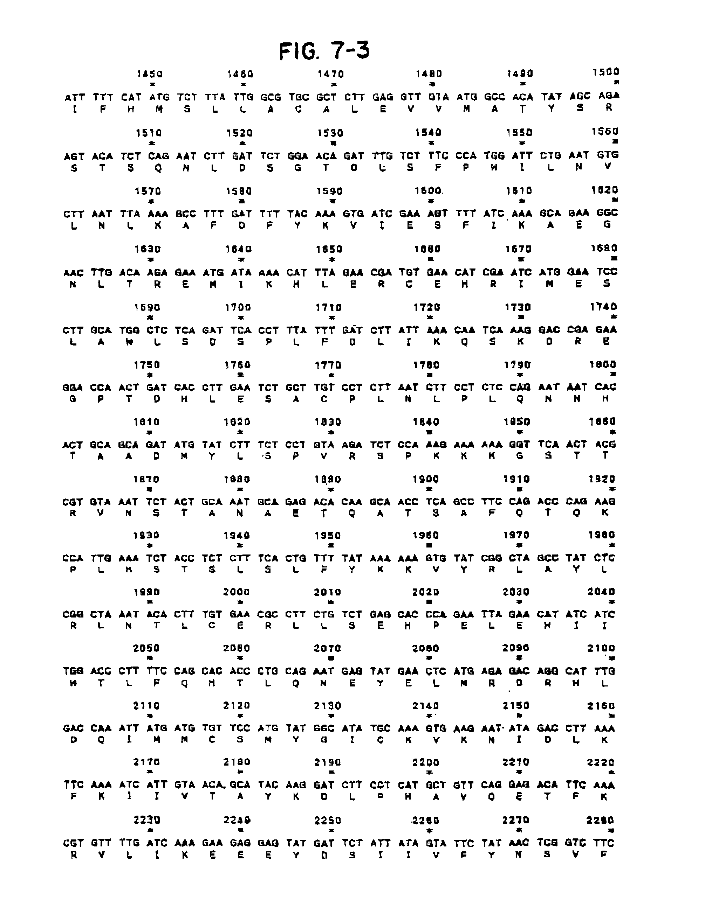

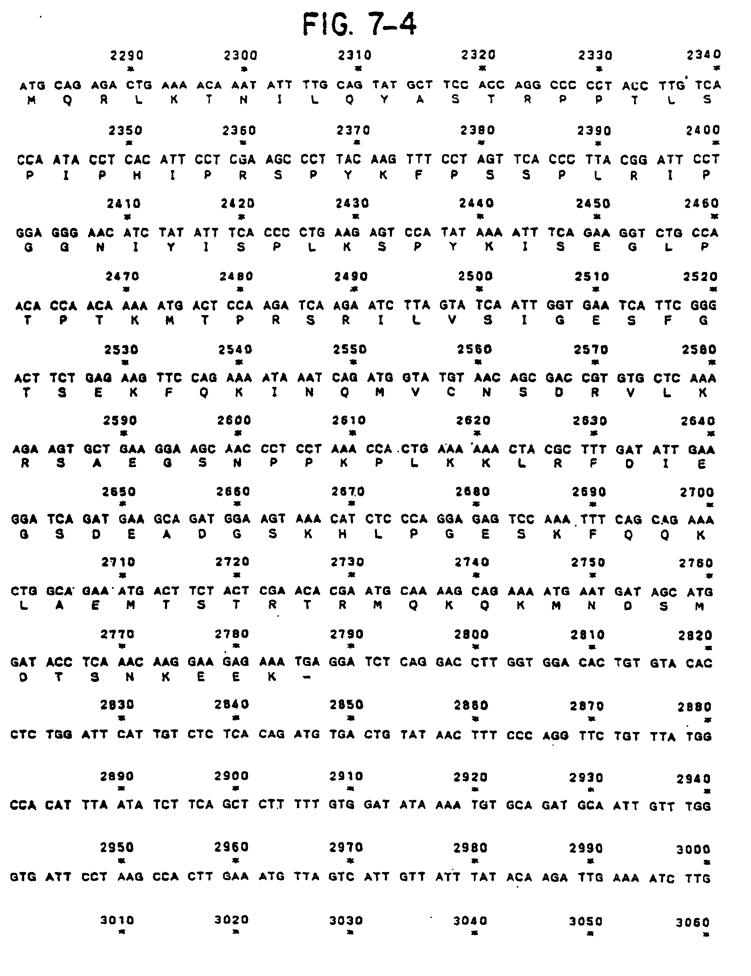

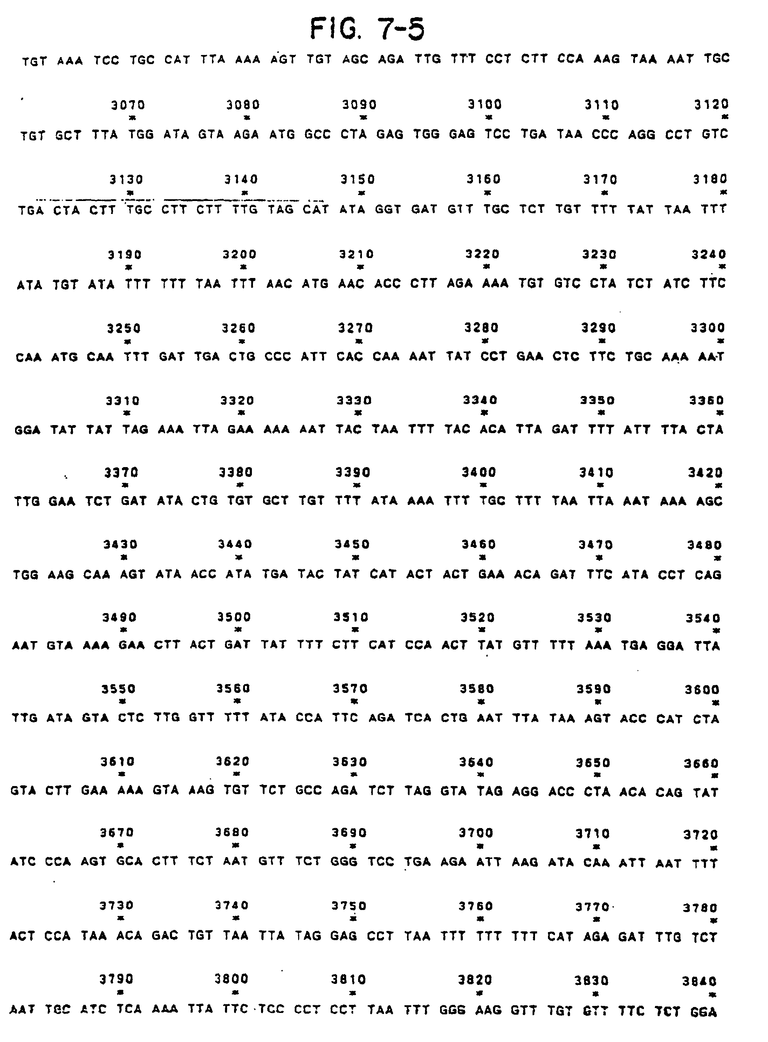

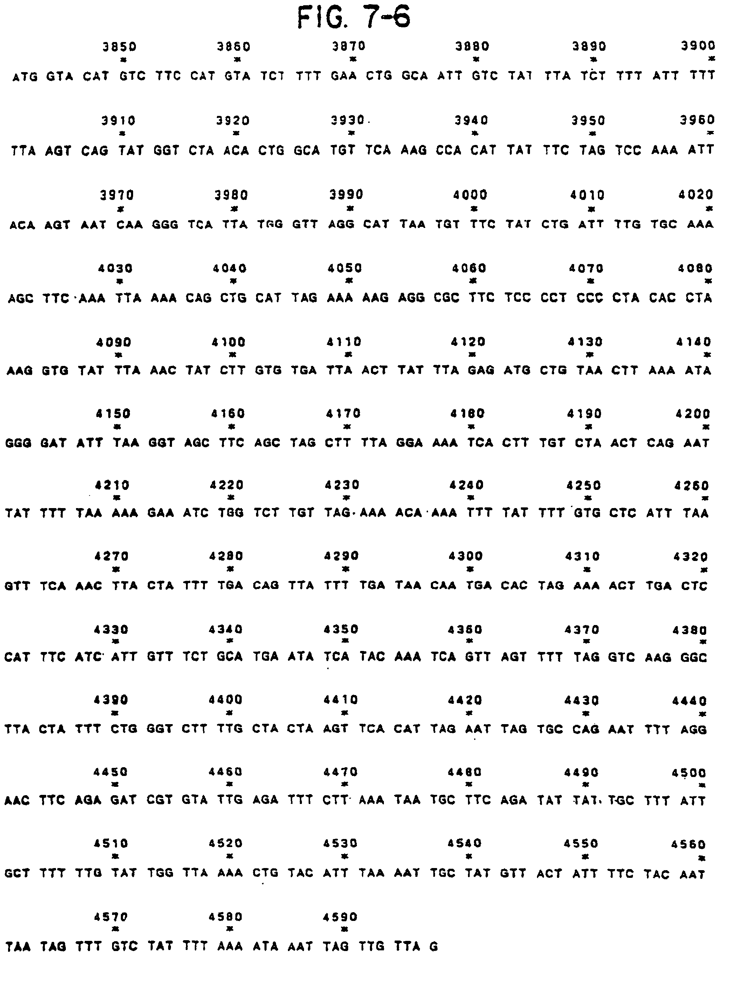

First, the nucleotide sequence of the normal Rb gene in p4.7R is determined by subcloning restriction fragments of ∼ 500 bp from p4.7R into an M13mp8 phage vector and sequencing these sublones by the dideoxy technique (Sanger et al., 1977, Proc. Nat. Acad. Sci USA 74:5463). A composite sequence of the Rb gene then can be assembled from these individual subclone sequences. This sequence is given in Fig. 7 which also shows flanking regions.

The isolated Rb gene alleles are sequenced according to the following procedure. Restriction fragments (- 2kb) of the allele are subcloned into the M13mp8 vector, and short stretches (∼500 bp) are sequenced individually using small restriction fragments isolated from p4.7R as the primers in the dideoxy sequencing reactions. The composite nucleotide sequence of the isolated allele then can be constructed from these individually-primed sequences. This sequence is compared directly with the sequence of the normal Rb gene, determined from p4.7R, to determine if any deletions or point mutations exist in the isolated allele.

### (3) Ribonuclease Cleavage of Mismatches

An alternative method of comparing the allelic DNA with the normal Rb gene employs RNase A to detect the existence of differences between the p4.7R sequence and the allele sequence. This comparison is performed in steps with small (∼ 500 bp) restriction fragments of p4.7R as the probe. First, p4.7R is digested with a restriction enzyme(s) that cuts the Rb gene sequence into fragments of approximately 500bp. These fragements are isolated on an electrophoresis gel and cloned individually, in both orientations, into an SP6 vector, such as pSP64 or pSP65 (Melton et al., 1984, Nucleic Acids Res. 12:7035). The SP6-based plasmids containing inserts of p4.7R fragments are transcribed in vitro using the SP6 transcription system, well known in the art, in the presence of [α-³²P]GTP, generating radiolabeled RNA transcripts of both strands of the cDNA of the Rb gene.

Individually, these RNA transcripts are used to form heteroduplexes with the allelic DNA, as follows. 50 ng of the allele subclone is digested with a restriction enzyme that cuts outside of the region covered by the RNA transcript probe to be used. This digested DNA is mixed with the radiolabeled RNA probe in 30 µl of hybrization buffer (80% formamide, 40 mM Pipes pH6.4, 0.4M NaCl, and 1mM EDTA) and the mixture is treated at 90°C for 10 minutes to denature the DNA. The mixture then is cooled slowly to 45°C and the RNA is allowed to anneal to the single-stranded DNA at 45°C for half an hour.

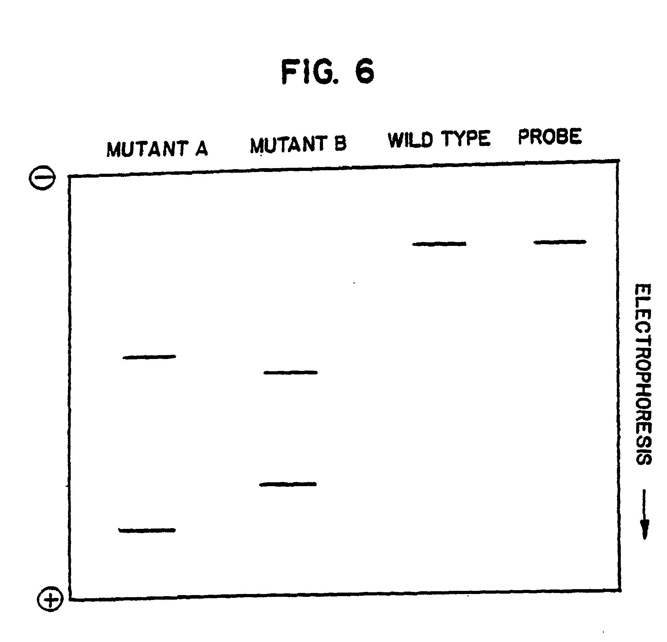

The RNA:DNA heteroduplexes next are treated with 350 µl of an RNase A solution (Sigma) (40 µg/ml in 10mM Tris-HCl pH7.5, 1mM EDTA, 0.2M NaCl, and 0.1M LiCl). The mixture is vortexed and incubated at 25°C for 30 minutes. The RNase A reaction is stopped by adding 10µl of proteinase K (10mg/ml) (Boehringer Mannheim) followed by incubation at 37°C for 20 minutes. Extraction with phenol-chloroform and ethanol precipitation of the aqueous layer yields a nucleic acid sample free from protein contamination. The precipitated sample is resuspended in 5µl and analyzed by denaturing polyacrylamide gel electrophoresis (4% polyacrylamide, 7M urea) (Fig. 5).

Mismatches that occur in the RNA:DNA heteroduplex, due to sequence differences between the p4.7R fragment and the Rb allele subclone from the individual, result in cleavage in the RNA strand by the RNase A treatment. Such mismatches can be the result of point mutations or small deletions in the individual's Rb allele. Cleavage of the RNA strand yields two or more small RNA fragments, which run faster on the denaturing gel than the RNA probe itself, as shown in Fig. 6.

In the above RNAse A technique, radiolabeled Rb gene RNA is hybridized to single strands of an individual's Rb allele which has been cloned into a vector. The RNase A technique is advantageous, however, because it also can be used without having to clone the Rb alleles. Preferably, genomic DNA is isolated from blood cells of the individual to be tested, and this genomic DNA is hybridized directly with the radiolabeled Rb RNA probes to determine sequence differences from the normal Rb gene, as follows. 5 µg of isolated, total genomic DNA is resuspended with the labeled RNA probe in 30 µl of hybridization buffer (80% formamide, 40mM Pipes pH6.4, 0.HM NaCl, and 1mM EDTA), and this hybridization mix is treated at 90°C for 10 minutes to denature the DNA. The mixture then is cooled slowly to 45°C and incubated at this temperature for 10 hours to allow hybridization of the RNA probe to the single-stranded DNA copies of the Rb allele. After hybridization, the RNase A treatment and electophoresis are performed as above. Mismatches in the heteroduplexes between the RNA probe and the genomic copies of the individual's Rb alleles are readily detected.

### Example 3: Protein Therapy

Another use for the cloned cDNA of the normal Rb gene, as represented by p4.7R, is to produce the Rb protein for treatment of individuals determined to carry a defective allele of the Rb gene. To prevent the formation of retinoblastoma in these individuals, the Rb gene product is administered therapeutically to these individuals. The Rb protein is produced by cloning the Rb cDNA from p4.7R into an appropriate mammalian expression vector, expressing the Rb protein from this vector in an in vivo expression system, and isolating the Rb protein from the medium or cells of the expression system. General in vitro expression vectors and systems are well known in the art.

### Example 4: Immunodiagnosis

The Rb protein, produced as described above, is injected into a rabbit to produce anti-Rb antibody, which then is labeled, e.g., radioactively, fluorescently, or with an enzyme such as alkaline phosphatase. The labeled antibody is used to determine whether human tumors are of defective Rb gene origin. This can be carried out using any conventional technique. For example, the tumor sample can be liquified and tested against the labeled antibody using a conventional ELISA format. Alternatively, a tumor section can be fixed and reacted with labeled antibody, and any immune complexes then can be detected by autoradiography or fluorescence microscopy, depending on the type of label on the antibody. Tumors lacking an antigen reactive with the antibody to the Rb gene product are due to mutations of the retinoblastoma gene. Since the tumors known to be caused by a mutant Rb gene are few (including retinoblastoma and osteosarcoma), the differential diagnosis of tumors deficient in the Rb gene product is greatly limited by such a test.

### Deposits

The plasmids p2AR3.8 and p2AR0.9 were deposited on July 17, 1986 with the American Type Culture Collection, Rockville, Maryland, and assigned ATCC accession numbers 40,241 and 40,242, respectively.

## Claims (Claims for the following Contracting State(s): BE, CH, LI, ES, FR, GB, GR, IT, LU, NL, SE)

1. A method of detecting the presence, in a tumour sample, of a protein the absence of which is associated with a neoplasm, the method comprising producing an antibody to the protein, contacting the antibody with the tumour sample, and detecting immune complexes as an indication of the presence in the tumour sample of the protein, provided that the protein is not a retinoblastoma protein.

2. Purified nucleic acid comprising a human retinoblastoma gene, or a unique subregion thereof, and further **characterized in that** said nucleic acid sequence is selected from the group consisting of:
(a) a nucleic acid sequence shown in Figure 7;
(b) a nucleic acid sequence capable of hybridizing specifically under hybridizing conditions to the nucleic acid sequence shown in Figure 7;
(c) a nucleic acid sequence capable of hybridizing specifically under hybridizing conditions to EcoRI-insert of plasmid p2AR3.8 (ATCC deposit number 40241); and
(d) a nucleic acid sequence capable of hybridizing specifically under hybridizing conditions to EcoRI-insert of plasmid p2AR0.9 (ATCC deposit number 40242), provided that the purified nucleic acid is not a vector comprising genetic material corresponding to a normal human retinoblastoma gene or a unique subregion thereof.

3. Purified nucleic acid according to Claim 2, further **characterized in that** the subregion is at least 20 base pairs in length.

4. Purified nucleic acid according to Claim 2, further **characterized in that** said nucleic acid hybridizes specifically to said retinoblastoma gene under hybridizing conditions.

5. Purified nucleic acid according to any of Claims 2 to 4, further **characterized in that** said nucleic acid has a restriction fragment map as shown in Figure 2.

6. A cell transformed with a nucleic acid according to any of Claims 2 to 5.

7. A retinoblastoma polypeptide comprising an amino acid sequence coded for by a nucleic acid according to any of Claims 2 to 5.

8. A retinoblastoma polypeptide according to Claim 7, further **characterized in that** said polypeptide is selected from the group consisting of: (a) an isolated naturally occurring retinoblastoma polypeptide; and (b) retinoblastoma polypeptide produced from purified retinoblastoma nucleic acid in an expression system.

9. A retinoblastoma polypeptide according to Claim 7, further **characterized in that** said polypeptide comprises an amino acid sequence shown in Figure 7.

10. A composition suitable for treating a human patient having a neoplasm resulting from a defective retinoblastoma allele, the composition **characterized in** comprising functional retinoblastoma polypeptide and a pharmacologically acceptable carrier.

11. Retinoblastoma polypeptide for use in treatment of a neoplasm resulting from a defective retinoblastoma allele.

12. Retinoblastoma polypeptide according to Claim 11 for use as specified therein, further **characterized in that** said neoplasm is a retinoblastoma.

13. Retinoblastoma polypeptide according to Claim 11 for use as specified therein, further **characterized in that** said neoplasm is an osteosarcoma.

14. Retinoblastoma polypeptide according to Claim 11 for use as specified therein, further **characterized in that** said neoplasm is an undifferentiated tumour.

15. An immunization-produced antibody capable of binding specifically to a retinoblastoma polypeptide according to any of Claims 7 to 9.

## Claims (Claims for the following Contracting State(s): DE)

1. A method of detecting the presence, in a tumour sample, of a protein the absence of which is associated with a neoplasm, the method comprising producing an antibody to the protein, contacting the antibody with the tumour sample, and detecting immune complexes as an indication of the presence in the tumour sample of the protein.

2. A method according to Claim 1, further **characterised in that** said protein is retinoblastoma protein.

3. A method according to Claim 2, further comprising the step of comparing a level of said immune complex that forms in said sample with a level of said immune complex that forms in a corresponding biological sample from a patient lacking said neoplasm and determining from said comparison whether said neoplasm is deficient in said functional form of said protein.

4. A method according to Claim 3, further **characterized in that** the level of said immune complex that forms in said sample is lower that the level of said immune complex that forms in a corresponding biological sample from a patient lacking said neoplasm.

5. Purified nucleic acid comprising a human retinoblastoma gene, or a unique subregion thereof and further **characterized in that** said nucleic acid sequence is selected from the group consisting of:
(a) a nucleic acid sequence shown in Figure 7;
(b) a nucleic acid sequence capable of hybridizing specifically under hybridizing conditions to the nucleic acid sequence shown in Figure 7;
(c) a nucleic acid sequence capable of hybridizing specifically under hybridizing conditions to EcoRI-insert of plasmid p2AR3.8 (ATCC deposit number 40241); and
(d) a nucleic acid sequence capable of hybridizing specifically under hybridizing conditions to EcoRI-insert of plasmid p2AR0.9 (ATCC deposit number 40242).

6. Purified nucleic acid according to Claim 5, further **characterized in that** the subregion is at least 20 base pairs in length.

7. Purified nucleic acid according to Claim 5, further **characterized in that** said nucleic acid hybridizes specifically to said retinoblastoma gene under hybridizing conditions.

8. Purified nucleic acid according to any of Claims 5 to 7, further **characterized in that** said nucleic acid has a restriction fragment map as shown in Figure 2.

9. A cell transformed with a nucleic acid according to any of Claims 5 to 8.

10. A retinoblastoma polypeptide comprising an amino acid sequence coded for by a nucleic acid according to any of Claims 5 to 8.

11. A retinoblastoma polypeptide according to Claim 10, further **characterized in that** said polypeptide is selected from the group consisting of: (a) an isolated naturally occurring retinoblastoma polypeptide; and (b) retinoblastoma polypeptide produced from purified retinoblastoma nucleic acid in an expression system.

12. A retinoblastoma polypeptide according to Claim 10, further **characterized in that** said polypeptide comprises an amino acid sequence shown in Figure 7.

13. A composition suitable for treating a human patient having a neoplasm resulting from a defective retinoblastoma allele, the composition **characterized in** comprising functional retinoblastoma polypeptide and a pharmacologically acceptable carrier.

14. Retinoblastoma polypeptide for use in treatment of a neoplasm resulting from a defective retinoblastoma allele.

15. Retinoblastoma polypeptide according to Claim 14 for use as specified therein, further **characterized in that** said neoplasm is a retinoblastoma.

16. Retinoblastoma polypeptide according to Claim 14 for use as specified therein, further **characterized in that** said neoplasm is an osteosarcoma.

17. Retinoblastoma polypeptide according to Claim 14 for use as specified therein, further **characterized in that** said neoplasm is an undifferentiated tumour.

18. An immunization-produced antibody capable of binding specifically to a retinoblastoma polypeptide according to any of Claims 10 to 12.

## Claims (Claims for the following Contracting State(s): AT)

1. A method of detecting the presence, in a tumour sample, of a protein the absence of which is associated with a neoplasm, the method comprising producing an antibody to the protein, contacting the antibody with the tumour sample, and detecting immune complexes as an indication of the presence in the tumour sample of the protein, provided that the protein is not a retinoblastoma protein.

2. Use of a purified nucleic acid comprising a human retinoblastoma gene, or a unique subregion thereof, and further **characterized in that** said nucleic acid sequence is selected from the group consisting of:
(a) a nucleic acid sequence shown in Figure 7;
(b) a nucleic acid sequence capable of hybridizing specifically under hybridizing conditions to the nucleic acid sequence shown in Figure 7;
(c) a nucleic acid sequence capable of hybridizing specifically under hybridizing conditions to EcoRI-insert of plasmid p2AR3.8 (ATCC deposit number 40241); and
(d) a nucleic acid sequence capable of hybridizing specifically under hybridizing conditions to EcoRI-insert of plasmid p2AR0.9 (ATCC deposit number 40242), in manufacture of a medicament for diagnosis of retinoblastoma, provided that the purified nucleic acid is not a vector comprising genetic material corresponding to a normal human retinoblastoma gene or a unique subregion thereof.

3. Use of the purified nucleic acid according to Claim 2, further **characterized in that** the subregion is at least 20 base pairs in length.

4. Use of the purified nucleic acid according to Claim 2, further **characterized in that** said nucleic acid hybridizes specifically to said retinoblastoma gene under hybridizing conditions.

5. Use of the purified nucleic acid according to any of Claims 2 to 4, further **characterized in that** said nucleic acid has a restriction fragment map as shown in Figure 2.

6. A method of making retinoblastoma polypeptide comprising expressing an amino acid sequence coded for by a nucleic acid comprising a human retinoblastoma gene or a unique subregion thereof, said nucleic acid being further **characterised in that** it is selected from the group consisting of:
(a) a nucleic acid sequence shown in Figure 7;
(b) a nucleic acid sequence capable of hybridizing specifically under hybridizing conditions to the nucleic acid sequence shown in Figure 7;
(c) a nucleic acid sequence capable of hybridizing specifically under hybridizing conditions to EcoRI-insert of plasmid p2AR3.8 (ATCC deposit number 40241); and
(d) a nucleic acid sequence capable of hybridizing specifically under hybridizing conditions to EcoRI-insert of plasmid p2AR0.9 (ATCC deposit number 40242).

7. A method according to Claim 6, further **characterized in that** said polypeptide is selected from the group consisting of: (a) an isolated naturally occurring retinoblastoma polypeptide; and (b) retinoblastoma polypeptide produced from purified retinoblastoma nucleic acid in an expression system.

8. A method according to Claim 6, further **characterized in that** said polypeptide comprises an amino acid sequence shown in Figure 7.

9. A method of making a composition suitable for treating a human patient having a neoplasm resulting from a defective retinoblastoma allele, the method **characterized by** combining functional retinoblastoma polypeptide and a pharmacologically acceptable carrier.

10. Use of retinoblastoma polypeptide in manufacture of a medicament for treatment of a neoplasm resulting from a defective retinoblastoma allele.

11. Use according to Claim 10 further **characterized in that** said neoplasm is a retinoblastoma.

12. Use according to Claim 10 further **characterized in that** said neoplasm is an osteosarcoma.

13. Use according to Claim 10 further **characterized in that** said neoplasm is an undifferentiated tumour.

14. A method of obtaining an antibody comprising obtaining an antibody that binds specifically to a retinoblastoma polypeptide.

15. A method according to Claim 14 wherein the retinoblastoma polypeptide is as defined in any of Claims 6 to 8.

## Patentansprüche (Patentansprüche für folgende(n) Vertragsstaat(en): BE, CH, LI, ES, FR, GB, GR, IT, LU, NL, SE)

1. Verfahren zum Detektieren der Anwesenheit eines Proteins in einer Tumorprobe, dessen Fehlen bzw. Abwesenheit mit einem Neoplasma assoziiert ist, das Verfahren umfassend das Erzeugen eines Antikörpers gegen das Protein, das In-Kontakt-bringen des Antikörpers mit der Tumorprobe und das Detektieren des Immunkomplexes als Anzeichen der Anwesenheit des Proteins in der Tumorprobe, mit der Maßgabe, dass das Protein kein Retinoblastom-Protein ist, mit der Maßgabe, dass das Protein kein Retinoblastom-Protein ist.

2. Gereinigte Nukleinsäure, umfassend ein humanes Retinoblastom-Gen oder einen einzigartigen Unterbereich desselben und zusätzlich **dadurch gekennzeichnet, dass** die Nukleinsäuresequenz ausgewählt ist aus der Gruppe, bestehend aus:
(a) einer Nukleinsäuresequenz, gezeigt in Figur 7;
(b) einer Nukleinsäuresequenz, die spezifisch unter Hybridisierungsbedingungen an die Nukleinsäuresequenz, gezeigt in Fig. 7, hybridisieren kann;
(c) einer Nukleinsäuresequenz, die spezifisch unter Hybridisierungsbedingungen an das EcoR1-Insert des Plasmids p2AR3.8 (ATCC-Hinterlegungsnummer 40241) hybridisieren kann; und
(d) einer Nukleinsäuresequenz, die spezifisch unter Hybridisierungsbedingungen an das EcoR1-Insert des Plasmids p2AR0.9 (ATCC-Hinterlegungsnummer 40242) hybridisieren kann, mit der Maßgabe, dass die gereinigte Nukleinsäure kein Vektor ist, der genetisches Material umfasst, das einem normalen humanen Retinoblastom-Gen oder einem einzigartigen Unterbereich desselben entspricht.

3. Gereinigte Nukleinsäure gemäß Anspruch 2, weiterhin **dadurch gekennzeichnet, dass** der Unterbereich mindestens 20 Basenpaare lang ist.

4. Gereinigte Nukleinsäure gemäß Anspruch 2, weiterhin **dadurch gekennzeichnet, dass** die Nukleinsäure spezifisch an das Retinoblastom-Gen unter Hybridisierungsbedingungen hybridisiert.

5. Gereinigte Nukleinsäure gemäß einem der Ansprüche 2-4, zusätzlich **dadurch gekennzeichnet, dass** die Nukleinsäure eine Restriktionskarte, wie in Fig. 2 gezeigt, aufweist.

6. Zelle, transformiert mit einer Nukleinsäure gemäß einem der Ansprüche 2-5.

7. Retinoblastom-Polypeptid, umfassend eine Aminosäuresequenz, codiert von einer Nukleinsäure nach einem der Ansprüche 2-5.

8. Retinoblastom-Polypeptid gemäß Anspruch 7, weiterhin **dadurch gekennzeichnet, dass** das Polypeptid ausgewählt ist aus der Gruppe, bestehend aus:
(a) einem isolierten, natürlich auftretenden Retinoblastom-Polypeptid; und
(b) einem Retinoblastom-Polypeptid, hergestellt aus einer gereinigten Retinoblastom-Nukleinsäure in einem Expressionssystem.

9. Retinoblastom-Polypeptid gemäß Anspruch 7, weiterhin **dadurch gekennzeichnet, dass** das Polypeptid eine Aminosäuresequenz, gezeigt in Fig. 7, umfasst.

10. Zubereitung, geeignet für die Behandlung eines menschlichen Patienten mit einem Neoplasma, das aus einem fehlerhaften Retinoblastom-Allel resultiert, die Zubereitung **dadurch gekennzeichnet, dass** sie ein funktionales Retinoblastom-Polypeptid und einen pharmakologisch annehmbaren Träger umfasst.

11. Retinoblastom-Polypeptid für die Verwendung bei der Behandlung eines Neoplasmas, resultierend aus einem fehlerhaften Retinoblastom-Allel.

12. Retinoblastom-Polypeptid gemäß Anspruch 11 für die Verwendung, wie darin angegeben, zusätzlich **dadurch gekennzeichnet, dass** das Neoplasma ein Retinoblastom ist.

13. Retinoblastom-Polypeptid gemäß Anspruch 11 für die Verwendung, wie darin angegeben, zusätzlich **dadurch gekennzeichnet, dass** das Neoplasma ein Osteosarcom ist.

14. Retinoblastom-Polypeptid gemäß Anspruch 11 für die Verwendung, wie darin angegeben, zusätzlich **dadurch gekennzeichnet, dass** das Neoplasma ein undifferenzierter Tumor ist.

15. Ein durch Immunisierung erzeugter Antikörper, der spezifisch an ein Retinoblastom-Polypeptid nach einem der Ansprüche 7-9 binden kann.

## Patentansprüche (Patentansprüche für folgende(n) Vertragsstaat(en): DE)

1. Verfahren zum Detektieren der Anwesenheit eines Proteins in einer Tumorprobe, dessen Fehlen bzw. Abwesenheit mit einem Neoplasma assoziiert ist, das Verfahren umfassend das Erzeugen eines Antikörpers gegen das Protein, das In-Kontakt-bringen des Antikörpers mit der Tumorprobe und das Detektieren von Immunkomplexen als Anzeichen der Anwesenheit des Proteins in der Tumorprobe.

2. Verfahren gemäß Anspruch 1, zusätzlich **gekennzeichnet dadurch, dass** das Protein ein Retinoblastom-Protein ist.

3. Verfahren gemäß Anspruch 2, zusätzlich umfassend den Schritt des Vergleichens eines Levels des Immunkomplexes, der sich in der Probe bildet, mit einem Level des Immunkomplexes, der sich in einer entsprechenden biologischen Probe aus einem Patienten ohne Neoplasma bilden und Bestimmen aus dem Vergleich, ob es dem Neoplasma an der funktionalen Form dieses Proteins mangelt.

4. Verfahren gemäß Anspruch 3, weiterhin **dadurch gekennzeichnet, dass** der Level des Immunkomplexes, der sich in der Probe bildet, niedriger als der Level des Immunkomplexes ist, der sich in einer entsprechenden biologischen Probe aus einem Patienten ohne Neoplasma bildet.

5. Gereinigte Nukleinsäure, umfassend ein humanes Retinoblastom-Gen oder einen einzigartigen Unterbereich desselben und zusätzlich **dadurch gekennzeichnet, dass** die Nukleinsäuresequenz ausgewählt ist aus der Gruppe, bestehend aus:
(a) einer Nukleinsäuresequenz, gezeigt in Figur 7;
(b) einer Nukleinsäuresequenz, die spezifisch unter Hybridisierungsbedingungen an die Nukleinsäuresequenz, gezeigt in Fig. 7, hybridisieren kann;
(c) einer Nukleinsäuresequenz, die spezifisch unter Hybridisierungsbedingungen an das EcoR1-Insert des Plasmids p2AR3.8 (ATCC-Hinterlegungsnummer 40241) hybridisieren kann; und
(d) einer Nukleinsäuresequenz, die spezifisch unter Hybridisierungsbedingungen an das EcoR1-Insert des Plasmids p2AR0.9 (ATCC-Hinterlegungsnummer 40242) hybridisieren kann.

6. Gereinigte Nukleinsäure gemäß Anspruch 5, weiterhin **dadurch gekennzeichnet, dass** der Unterbereich mindestens 20 Basenpaare lang ist.

7. Gereinigte Nukleinsäure gemäß Anspruch 5, weiterhin **dadurch gekennzeichnet, dass** die Nukleinsäure spezifisch an das Retinoblastom-Gen unter Hybridisierungsbedingungen hybridisiert.

8. Gereinigte Nukleinsäure gemäß einem der Ansprüche 5-7, zusätzlich **dadurch gekennzeichnet, dass** die Nukleinsäure eine Restriktionskarte, wie in Fig. 2 gezeigt, aufweist.

9. Zelle, transformiert mit einer Nukleinsäure gemäß einem der Ansprüche 5-8.

10. Retinoblastom-Polypeptid, umfassend eine Aminosäuresequenz, codiert von einer Nukleinsäure nach einem der Ansprüche 5-8.

11. Retinoblastom-Polypeptid gemäß Anspruch 10, weiterhin **dadurch gekennzeichnet, dass** das Polypeptid ausgewählt ist aus der Gruppe, bestehend aus:
(a) einem isolierten, natürlich auftretenden Retinoblastom-Polypeptid; und
(b) einem Retinoblastom-Polypeptid, hergestellt von einer gereinigten Retinoblastom-Nukleinsäure in einem Expressionssystem.

12. Retinoblastom-Polypeptid gemäß Anspruch 10, weiterhin **dadurch gekennzeichnet, dass** das Polypeptid eine Aminosäuresequenz, gezeigt in Fig. 7, umfasst.

13. Zubereitung, geeignet für die Behandlung eines menschlichen Patienten mit einem Neoplasma, das aus einem fehlerhaften Retinoblastom-Allel resultiert, die Zubereitung **dadurch gekennzeichnet, dass** sie ein funktionales Retinoblastom-Polypeptid und einen pharmakologisch annehmbaren Träger umfasst.

14. Retinoblastom-Polypeptid für die Verwendung bei der Behandlung eines Neoplasmas, resultierend aus einem fehlerhaften Retinoblastom-Allel

15. Retinoblastom-Polypeptid gemäß Anspruch 14 für die Verwendung, wie darin angegeben, zusätzlich **dadurch gekennzeichnet, dass** das Neoplasma ein Retinoblastom ist.

16. Retinoblastom-Polypeptid gemäß Anspruch 14 für die Verwendung, wie darin angegeben, zusätzlich **dadurch gekennzeichnet, dass** das Neoplasma ein Osteosarcom ist.

17. Retinoblastom-Polypeptid gemäß Anspruch 14 für die Verwendung, wie darin angegeben, zusätzlich **dadurch gekennzeichnet, dass** das Neoplasma ein undifferenzierter Tumor ist.

18. Ein durch Immunisierung erzeugter Antikörper, der spezifisch an ein Retinoblastom-Polypeptid nach einem der Ansprüche 10-12 binden kann.

## Patentansprüche (Patentansprüche für folgende(n) Vertragsstaat(en): AT)

1. Verfahren zum Detektieren der Anwesenheit eines Proteins in einer Tumorprobe, dessen Fehlen bzw. Abwesenheit mit einem Neoplasma assoziiert ist, das Verfahren umfassend das Erzeugen eines Antikörpers gegen das Protein, das In-Kontakt-bringen des Antikörpers mit der Tumorprobe und das Detektieren des Immunkomplexes als Anzeichen der Anwesenheit des Proteins in der Tumorprobe, mit der Maßgabe, dass das Protein kein Retinoblastom-Protein ist.

2. Verwendung einer gereinigten Nukleinsäure, umfassend ein humanes Retinoblastom-Gen oder einen einzigartigen Unterbereich desselben und zusätzlich **dadurch gekennzeichnet, dass** die Nukleinsäuresequenz ausgewählt ist aus der Gruppe, bestehend aus:
(a) einer Nukleinsäuresequenz, gezeigt in Figur 7;
(b) einer Nukleinsäuresequenz, die spezifisch unter Hybridisierungsbedingungen an die Nukleinsäuresequenz, gezeigt in Fig. 7, hybridisieren kann;
(c) einer Nukleinsäuresequenz, die spezifisch unter Hybridisierungsbedingungen an das EcoR1-Insert des Plasmids p2AR3.8 (ATCC-Hinterlegungsnummer 40241) hybridisieren kann;
(d) einer Nukleinsäuresequenz, die spezifisch unter Hybridisierungsbedingungen an das EcoR1-Insert des Plasmids p2AR0.9 (ATCC-Hinterlegungsnummer 40242) hybridisieren kann, bei der Herstellung eines Medikaments für die Diagnose eines Retinoblastoms, mit der Maßgabe, dass die gereinigte Nukleinsäure kein Vektor ist, der genetisches Material umfasst, das einem normalen humanen Retinoblastom-Gen oder einem einzigartigen Unterbereich desselben entspricht.

3. Verwendung einer gereinigten Nukleinsäure gemäß Anspruch 2, weiterhin **dadurch gekennzeichnet, dass** der Unterbereich mindestens 20 Basenpaare lang ist.

4. Verwendung einer gereinigten Nukleinsäure gemäß Anspruch 2, weiterhin **dadurch gekennzeichnet, dass** die Nukleinsäure spezifisch an das Retinoblastom-Gen unter Hybridisierungsbedingungen hybridisiert.

5. Verwendung einer gereinigten Nukleinsäure gemäß einem der Ansprüche 2-4, zusätzlich **dadurch gekennzeichnet, dass** die Nukleinsäure eine Restriktionskarte, wie in Fig. 2 gezeigt, aufweist.

6. Verfahren der Herstellung eines Retinoblastom-Polypeptids, umfassend das Exprimieren einer Aminosäuresequenz, codiert von einer Nukleinsäure, umfassend ein humanes Retinoblastom-Gen oder einen einzigartigen Unterbereich desselben, wobei die Nukleinsäure zusätzlich **dadurch gekennzeichnet ist, dass** sie ausgewählt ist aus der Gruppe, bestehend aus:
(a) einer Nukleinsäuresequenz, gezeigt in Figur 7;
(b) einer Nukleinsäuresequenz, die spezifisch unter Hybridisierungsbedingungen an die Nukleinsäuresequenz, gezeigt in Fig. 7, hybridisieren kann;
(c) einer Nukleinsäuresequenz, die spezifisch unter Hybridisierungsbedingungen an das EcoR1-Insert des Plasmids p2AR3.8 (ATCC-Hinterlegungsnummer 40241) hybridisieren kann; und
(d) einer Nukleinsäuresequenz, die spezifisch unter Hybridisierungsbedingungen an das EcoR1-Insert des Plasmids p2AR0.9 (ATCC-Hinterlegungsnummer 40242) hybridisieren kann.

7. Verfahren gemäß Anspruch 6, weiterhin **dadurch gekennzeichnet, dass** das Polypeptid ausgewählt ist aus der Gruppe, bestehend aus:
(a) einem isolierten, natürlich auftretenden Retinoblastom-Polypeptid; und
(b) einem Retinoblastom-Polypeptid, hergestellt von einer gereinigten Retinoblastom-Nukleinsäure in einem Expressionssystem.

8. Verfahren gemäß Anspruch 6, weiterhin **dadurch gekennzeichnet, dass** das Polypeptid eine Aminosäuresequenz, gezeigt in Fig. 7, umfasst.

9. Verfahren zur Herstellung einer Zubereitung, geeignet für die Behandlung eines menschlichen Patienten mit einem Neoplasma, das aus einem fehlerhaften Retinoblastom-Allel resultiert, das Verfahren **dadurch gekennzeichnet, dass** man ein funktionales Retinoblastom-Polypeptid und einen pharmakologisch annehmbaren Träger kombiniert.

10. Verwendung eines Retinoblastom-Polypeptids für die Herstellung eines Medikaments zur Behandlung eines Neoplasmas, resultierend aus einem fehlerhaften Retinoblastom-Allel.

11. Verwendung gemäß Anspruch 10 für die Verwendung wie darin angegeben, zusätzlich **dadurch gekennzeichnet, dass** das Neoplasma ein Retinoblastom ist.

12. Verwendung gemäß Anspruch 10 **dadurch gekennzeichnet, dass** das Neoplasma ein Osteosarcom ist.

13. Verwendung gemäß Anspruch 10 **dadurch gekennzeichnet, dass** das Neoplasma ein undifferenzierter Tumor ist.

14. Verfahren zum Erhalt eines Antikörpers, umfassend den Erhalt eines Antikörpers, der spezifisch an ein Retinoblastom-Polypeptid bindet.

15. Verfahren gemäß Anspruch 14, worin das Retinoblastom-Polypeptid wie in einem der Ansprüche 6-8 definiert ist.

## Revendications (Revendications pour l'(les) Etat(s) contractant(s) suivant(s): BE, CH, LI, ES, FR, GB, GR, IT, LU, NL, SE)

1. Procédé pour détecter la présence, dans un échantillon de tumeur, d'une protéine dont l'absence est associée à un néoplasme, le procédé comprenant la production d'un anticorps dirigé contre la protéine, la mise en contact de l'anticorps avec l'échantillon de tumeur, et la détection de complexes immuns comme indication de la présence de la protéine dans l'échantillon de tumeur, à condition que la protéine ne soit pas une protéine de rétinoblastome.

2. Acide nucléique purifié comprenant un gène de rétinoblastome humain, ou une sous-région unique de celui-ci, et **caractérisé en outre en ce que** la séquence dudit acide nucléique est choisie dans le groupe consistant en :
(a) une séquence d'acide nucléique montrée sur la figure 7 ;
(b) une séquence d'acide nucléique capable de s'hybrider spécifiquement dans des conditions d'hybridation à la séquence d'acide nucléique montrée sur la figure 7 ;
(c) une séquence d'acide nucléique capable de s'hybrider spécifiquement dans des conditions d'hybridation à l'insert EcoRI du plasmide p2AR3.8 (numéro de dépôt ATCC 40241) ; et
(d) une séquence d'acide nucléique capable de s'hybrider spécifiquement dans des conditions d'hybridation à l'insert EcoRI du plasmide p2AR0.9 (numéro de dépôt ATCC 40242), à condition que l'acide nucléique purifié ne soit pas un vecteur comprenant un matériel génétique correspondant à un gène de rétinoblastome humain normal ou une sous-région unique de celui-ci.

3. Acide nucléique purifié selon la revendication 2 **caractérisé en outre en ce que** la sous-région est longue d'au moins 20 paires de bases.

4. Acide nucléique purifié selon la revendication 2 **caractérisé en outre en ce que** ledit acide nucléique s'hybride spécifiquement audit gène de rétinoblastome dans des conditions d'hybridation.

5. Acide nucléique purifié selon l'une quelconque des revendications 2 à 4 **caractérisé en outre en ce que** ledit acide nucléique a une carte de fragments de restriction telle que montrée sur la figure 2.

6. Cellule transformée avec un acide nucléique selon l'une quelconque des revendications 2 à 5.

7. Polypeptide de rétinoblastome comprenant une séquence d'acides aminés codée par un acide nucléique selon l'une quelconque des revendications 2 à 5.

8. Polypeptide de rétinoblastome selon la revendication 7 **caractérisé en outre en ce que** ledit polypeptide est choisi dans le groupe consistant en :
(a) un polypeptide de rétinoblastome naturel isolé ; et
(b) un polypeptide de rétinoblastome produit à partir d'un acide nucléique de rétinoblastome purifié dans un système d'expression.

9. Polypeptide de rétinoblastome selon la revendication 7 **caractérisé en outre en ce que** ledit polypeptide comprend une séquence d'acides aminés montrée sur la figure 7.

10. Composition convenant pour traiter un patient humain ayant un néoplasme résultant d'un allèle de rétinoblastome défectueux, la composition étant **caractérisée en ce qu'**elle comprend un polypeptide de rétinoblastome fonctionnel et un support pharmacologiquement acceptable.

11. Polypeptide de rétinoblastome destiné à être utilisé dans le traitement d'un néoplasme résultant d'un allèle de rétinoblastome défectueux.

12. Polypeptide de rétinoblastome selon la revendication 11 destiné à être utilisé de la manière spécifiée, **caractérisé en outre en ce que** ledit néoplasme est un rétinoblastome.

13. Polypeptide de rétinoblastome selon la revendication 11 destiné à être utilisé de la manière spécifiée, **caractérisé en outre en ce que** ledit néoplasme est un ostéosarcome.

14. Polypeptide de rétinoblastome selon la revendication 11 destiné à être utilisé de la manière spécifiée, **caractérisé en outre en ce que** ledit néoplasme est une tumeur indifférenciée.

15. Anticorps produit par immunisation capable de se lier spécifiquement à un polypeptide de rétinoblastome selon l'une quelconque des revendications 7 à 9.

## Revendications (Revendications pour l'(les) Etat(s) contractant(s) suivant(s): DE)

1. Procédé pour détecter la présence, dans un échantillon de tumeur, d'une protéine dont l'absence est associée à un néoplasme, le procédé comprenant la production d'un anticorps dirigé contre la protéine, la mise en contact de l'anticorps avec l'échantillon de tumeur, et la détection de complexes immuns comme indication de la présence de la protéine dans l'échantillon de tumeur.

2. Procédé selon la revendication 1 **caractérisé en outre en ce que** ladite protéine est une protéine de rétinoblastome.

3. Procédé selon la revendication 2 comprenant en outre l'étape de comparaison d'un niveau dudit complexe immun qui se forme dans ledit échantillon avec un niveau dudit complexe immun qui se forme dans un échantillon biologique correspondant provenant d'un patient dépourvu dudit néoplasme et de détermination d'après ladite comparaison de ce que ledit néoplasme est déficient concernant ladite forme fonctionnelle de ladite protéine.

4. Procédé selon la revendication 3 **caractérisé en outre en ce que** le niveau dudit complexe immun qui se forme dans ledit échantillon est inférieur au niveau dudit complexe immun qui se forme dans un échantillon biologique correspondant provenant d'un patient dépourvu dudit néoplasme.

5. Acide nucléique purifié comprenant un gène de rétinoblastome humain, ou une sous-région unique de celui-ci, et **caractérisé en outre en ce que** la séquence dudit acide nucléique est choisie dans le groupe consistant en :
(a) une séquence d'acide nucléique montrée sur la figure 7 ;
(b) une séquence d'acide nucléique capable de s'hybrider spécifiquement dans des conditions d'hybridation à la séquence d'acide nucléique montrée sur la figure 7 ;
(c) une séquence d'acide nucléique capable de s'hybrider spécifiquement dans des conditions d'hybridation à l'insert EcoRI du plasmide p2AR3.8 (numéro de dépôt ATCC 40241) ; et
(d) une séquence d'acide nucléique capable de s'hybrider spécifiquement dans des conditions d'hybridation à l'insert EcoRI du plasmide p2AR0.9 (numéro de dépôt ATCC 40242).

6. Acide nucléique purifié selon la revendication 5 **caractérisé en outre en ce que** la sous-région est longue d'au moins 20 paires de bases.

7. Acide nucléique purifié selon la revendication 5 **caractérisé en outre en ce que** ledit acide nucléique s'hybride spécifiquement audit gène de rétinoblastome dans des conditions d'hybridation.

8. Acide nucléique purifié selon l'une quelconque des revendications 5 à 7 **caractérisé en outre en ce que** ledit acide nucléique a une carte de fragments de restriction telle que montrée sur la figure 2.

9. Cellule transformée avec un acide nucléique selon l'une quelconque des revendications 5 à 8.

10. Polypeptide de rétinoblastome comprenant une séquence d'acides aminés codée par un acide nucléique selon l'une quelconque des revendications 5 à 8.

11. Polypeptide de rétinoblastome selon la revendication 10 **caractérisé en outre en ce que** ledit polypeptide est choisi dans le groupe consistant en :
(a) un polypeptide de rétinoblastome naturel isolé ; et
(b) un polypeptide de rétinoblastome produit à partir d'un acide nucléique de rétinoblastome purifié dans un système d'expression.

12. Polypeptide de rétinoblastome selon la revendication 10 **caractérisé en outre en ce que** ledit polypeptide comprend une séquence d'acides aminés montrée sur la figure 7.

13. Composition convenant pour traiter un patient humain ayant un néoplasme résultant d'un allèle de rétinoblastome défectueux, la composition étant **caractérisée en ce qu'**elle comprend un polypeptide de rétinoblastome fonctionnel et un support pharmacologiquement acceptable.

14. Polypeptide de rétinoblastome destiné à être utilisé dans le traitement d'un néoplasme résultant d'un allèle de rétinoblastome défectueux.

15. Polypeptide de rétinoblastome selon la revendication 14 destiné à être utilisé de la manière spécifiée ici, **caractérisé en outre en ce que** ledit néoplasme est un rétinoblastome.

16. Polypeptide de rétinoblastome selon la revendication 14 destiné à être utilisé de la manière spécifiée ici, **caractérisé en outre en ce que** ledit néoplasme est un ostéosarcome.

17. Polypeptide de rétinoblastome selon la revendication 14 destiné à être utilisé de la manière spécifiée ici, **caractérisé en outre en ce que** ledit néoplasme est une tumeur indifférenciée.

18. Anticorps produit par immunisation capable de se lier spécifiquement à un polypeptide de rétinoblastome selon l'une quelconque des revendications 10 à 12.

## Revendications (Revendications pour l'(les) Etat(s) contractant(s) suivant(s): AT)

1. Procédé pour détecter la présence, dans un échantillon de tumeur, d'une protéine dont l'absence est associée à un néoplasme, le procédé comprenant la production d'un anticorps dirigé contre la protéine, la mise en contact de l'anticorps avec l'échantillon de tumeur, et la détection de complexes immuns comme indication de la présence de la protéine dans l'échantillon de tumeur, à condition que la protéine ne soit pas une protéine de rétinoblastome.

2. Utilisation d'un acide nucléique purifié comprenant un gène de rétinoblastome humain, ou une sous-région unique de celui-ci, et **caractérisée en outre en ce que** la séquence dudit acide nucléique est choisie dans le groupe consistant en :
(a) une séquence d'acide nucléique montrée sur la figure 7 ;
(b) une séquence d'acide nucléique capable de s'hybrider spécifiquement dans des conditions d'hybridation à la séquence d'acide nucléique montrée sur la figure 7 ;
(c) une séquence d'acide nucléique capable de s'hybrider spécifiquement dans des conditions d'hybridation à l'insert EcoRI du plasmide p2AR3.8 (numéro de dépôt ATCC 40241) ; et
(d) une séquence d'acide nucléique capable de s'hybrider spécifiquement dans des conditions d'hybridation à l'insert EcoRI du plasmide p2AR0.9 (numéro de dépôt ATCC 40242), dans la fabrication d'un médicament pour le diagnostic du rétinoblastome, à condition que l'acide nucléique purifié ne soit pas un vecteur comprenant un matériel génétique correspondant à un gène de rétinoblastome humain normal ou une sous-région unique de celui-ci.

3. Utilisation de l'acide nucléique purifié selon la revendication 2 **caractérisée en outre en ce que** la sous-région est longue d'au moins 20 paires de bases.

4. Utilisation de l'acide nucléique purifié selon la revendication 2 **caractérisée en outre en ce que** ledit acide nucléique s'hybride spécifiquement audit gène de rétinoblastome dans des conditions d'hybridation.

5. Utilisation de l'acide nucléique purifié selon l'une quelconque des revendications 2 à 4 **caractérisée en outre en ce que** ledit acide nucléique a une carte de fragments de restriction telle que montrée sur la figure 2.

6. Procédé de production d'un polypeptide de rétinoblastome comprenant l'expression d'une séquence d'acides aminés codée par un acide nucléique comprenant un gène de rétinoblastome humain, ou une sous-région unique de celui-ci, ledit acide nucléique étant **caractérisé en outre en ce qu'**il est choisi dans le groupe consistant en :
(a) une séquence d'acide nucléique montrée sur la figure 7 ;
(b) une séquence d'acide nucléique capable de s'hybrider spécifiquement dans des conditions d'hybridation à la séquence d'acide nucléique montrée sur la figure 7 ;
(c) une séquence d'acide nucléique capable de s'hybrider spécifiquement dans des conditions d'hybridation à l'insert EcoRI du plasmide p2AR3.8 (numéro de dépôt ATCC 40241) ; et
(d) une séquence d'acide nucléique capable de s'hybrider spécifiquement dans des conditions. d'hybridation à l'insert EcoRI du plasmide p2AR0.9 (numéro de dépôt ATCC 40242).

7. Procédé selon la revendication 6 **caractérisé en outre en ce que** ledit polypeptide est choisi dans le groupe consistant en : (a) un polypeptide de rétinoblastome naturel isolé ; et (b) un polypeptide de rétinoblastome produit à partir d'un acide nucléique de rétinoblastome purifié dans un système d'expression.

8. Procédé selon la revendication 6 **caractérisé en outre en ce que** ledit polypeptide comprend une séquence d'acides aminés montrée sur la figure 7.

9. Procédé de production d'une composition convenant pour traiter un patient humain ayant un néoplasme résultant d'un allèle de rétinoblastome défectueux, le procédé étant **caractérisé par** la combinaison d'un polypeptide de rétinoblastome fonctionnel et d'un support pharmacologiquement acceptable.

10. Utilisation d'un polypeptide de rétinoblastome dans la fabrication d'un médicament pour le traitement d'un néoplasme résultant d'un allèle de rétinoblastome défectueux.

11. Utilisation selon la revendication 10 **caractérisée en outre en ce que** ledit néoplasme est un rétinoblastome.

12. Utilisation selon la revendication 10 **caractérisée en outre en ce que** ledit néoplasme est un ostéosarcome.

13. Utilisation selon la revendication 10 **caractérisée en outre en ce que** ledit néoplasme est une tumeur indifférenciée.

14. Procédé d'obtention d'un anticorps comprenant l'obtention d'un anticorps qui se lie spécifiquement à un polypeptide de rétinoblastome.

15. Procédé selon la revendication 14 où le polypeptide de rétinoblastome est tel que défini dans l'une quelconque des revendications 6 à 8.
